# EUROPEAN PATENT SPECIFICATION

(11) **EP 2 049 651 B1**
(45) Date of publication and mention of the grant of the patent: **14.10.2015**
(21) Application number: 07789137.2
(22) Date of filing: 07.08.2007
(51) Int. Cl.: C12M 1/34

(54) **GAS EXCHANGE DETECTION METHOD**
GASAUSTAUSCHNACHWEISVERFAHREN
PROCÉDÉ DE DÉTECTION D'ÉCHANGE GAZEUX

(30) Priority: 08.08.2006 GB 0615678; 08.08.2006 US 836262 P
(43) Date of publication of application: 22.04.2009
(73) Proprietor: Metrica.bio Limited, Halstead CO9 4PD (GB)
(72) Inventor: FALL, Martyn, John, Essex CO9 4PD (GB); GROVES, Jan, Lowery, Chelmsford, Essex CM3 1QF (GB); HALL, Elizabeth, Ann, Howlett, Hampdhire SO21 3PG (GB); RUTHERFORD, James, Ormiston, Cambridge CB1 7RU (GB)
(74) Representative: Gibson, Mark
(86) International application number: PCT/GB2007/003002
(87) International publication number: WO 2008/017833

(56) References cited:
- EP-A- 0 592 728
- EP-A- 0 611 821
- WO-A-01/40435
- WO-A-92/01064
- WO-A-03/078563
- US-A- 3 764 508
- US-A- 4 152 213
- US-A- 5 051 360

## Description

The invention relates to a gas exchange detection method for rapid detection of the presence of micro-organisms, the effect of an additional substance upon a microorganism and/or the rate at which oxygen is consumed, in solutions, aqueous emulsions and/or suspensions.

In many fields, it is important to be able to determine whether materials are contaminated with micro-organisms. For example, in the medical field, micro-organisms can cause serious infection, which can be life threatening if not detected in a timely fashion. Furthermore, in a number of industrial areas, such as the food, cosmetic and sewage industries, detection of microbiological contamination or toxicity is of the utmost importance.

Whilst there are a number of methods that can be used to detect microorganisms, for example colony count enumeration, bioluminescence and impedimetry, recent studies suggest that short-term respirometry is an easier, more rapid and reliable methodology (M Simões et al.: Biofouling, 2005; 21(1): 9-17).

Although, a number of techniques are presently available for measuring gas production of micro-organisms, each has a number of disadvantages or limitations.

EP 0611821 (Commissariat a L'Energie Atomique) describes a process and device that determines biological activity by growing two cultures of micro-organisms in separate containers and comparing the pressure difference between the containers after a suitable length of time. However, the pressures are measured independently and the pressure within the first container must reach a pre-determined threshold before measurement of the pressure difference can be achieved. Sensitivity is also described as being as high as 25 x 10² Pa.

WO 03/078563 (Bactest Limited) describes an indirect pressure sensing system for non-invasive monitoring of gas exchange by biological material. However, this arrangement provides indirect pressure measurement, for example, pressure in a first container is measured by sensing pressure in a second container, which is sealed and isolated from the first container by a flexible diaphragm.

US3764508 describes an electrochemical oxygen demand measuring system having a pair of test cells, each of which contains a pair of electrodes immersed in the fluid of the cell.

It is therefore an object of the invention to provide a method which achieves a rapid measurement of microbiological activity and is sufficiently sensitive to detect low levels of microbiological activity without the need for a cultivating or concentration step.

Thus, according to a first aspect of the invention, there is provided a method for determining the presence of micro-organisms in a test sample and/or monitoring the effect of an additional substance upon a micro-organism which comprises the steps of:
(a) adding a reference sample to a receptacle within a reference chamber, wherein said reference sample has a known level of micro-organism activity;
(b) adding the test sample to a receptacle within a test chamber, wherein said test sample has an unknown level of micro-organism activity;
(c) sealing the chambers from the external environment; and
(d) directly and continuously monitoring the pressure differential between each of said chambers by taking multiple measurements, such as 1/second, over a specific time period,
wherein said method additionally comprises one or more parameter adjustment steps, such as temperature and/or mixing adjustment, prior to step (d).

The method of the invention is capable of monitoring gas uptake, evolution or exchange of a sample comprising biological or chemical components at extremely low levels by continuously monitoring the pressure difference between the reference chamber and test chamber and the rate at which it occurs. The increased sensitivity removes the need for a cultivating or concentration step, which helps to speed up the preparation of the test, and enables the method to achieve detection nearly 600 times faster than traditional microbiological culturing.

Furthermore, capturing multiple measurements of pressure offers numerous advantages when compared with taking one measurement. For example, the invention can provide the user with significantly more information with respect to the sample's components, such as the time required to modify a bacteria culture's respiration, the uptake of oxygen prior to release of CO₂, inhibition or enhancement of respiration following addition of biocide, antibiotic or nutrient etc.

It will also be appreciated that the simultaneous use of the reference chamber containing a reference (or control sample) having a similar matrix to the test sample helps to eliminate any errors resulting from variations in conditions that may occur if the samples were analysed separately.

In one embodiment, each chamber is partially filled with a sample comprising components capable of gas exchange and, therefore, of producing a pressure variation in the chamber's headspace. The components may be biological, e.g. respiring bacteria. Where the components are biological, pressure changes are a consequence of oxygen depletion caused by the respiring living organisms in the sample or release of a gas such as, but not exclusively carbon dioxide.

In one embodiment, the test sample is a sample with an unknown level of sterility or micro-organism activity, whilst the reference sample contains a reference (or control) sample, having a similar matrix to the test sample, but with a known level of sterility or micro-organism activity. In one embodiment, the reference sample may be free of micro-organisms (e.g. the sample may be inactive or sterile).

Pre-treatment of samples is usually un-necessary in steps (a) and (b), unless activity levels are so high that they are outside the pressure measuring range of the device. If this is the case, then aqueous dilution is required to bring the sample within the measuring range of the device. The sealing step (c) is necessary to achieve gas-tight chambers and is generally achieved by providing sealing means (e.g. a lid and o-ring) over each of the chambers.

In one embodiment, the parameter adjustment step comprises temperature adjustment. The activity of any given component (e.g. a micro-organism) will typically vary with temperature and each component typically has an optimum temperature. In this embodiment, the parameter adjustment step may require a pre-heating step to an optimum and/or constant temperature. An incubation step may also be required to enable the components of the sample to move from semi-dormancy to a more active state once an optimum temperature has been reached. The incubation step may also assist with removal of dissolved gases from the solution, which may mask any pressure change due to bacterial respiration. In one embodiment, the incubation step has a duration of between 1 and 6000 seconds, suitably between 10 and 600 seconds.

In one embodiment, the parameter adjustment step comprises pressure adjustment. In this embodiment, the pressure sensor is zeroed by opening exhaust valves and equalising the pressure in each chamber. In this embodiment, the parameter adjustment step may require a venting step to allow equilibration of the internal pressure of the chamber. In one embodiment, the venting step has a duration of between 5 and 60 seconds.

In one embodiment, the parameter adjustment step comprises mixing adjustment. For example, generally the rate and length of mixing affects gas exchange. In one embodiment, mixing occurs throughout pressure measurement step (d). In an alternative embodiment, mixing occurs intermittently.

In one embodiment, the parameter adjustment step may be conducted by processing means as defined herein (e.g. use of a set-up menu). Once optimised for a particular component, the parameters can be stored as an automatic setting. A menu-based system allows for the optimal setting of conditions for a wide variety of samples.

Once the parameters have been adjusted, the relative pressure change differences between the two chambers in step (d) is measured. Numerous measurements are made, such as 1/second, over a specific measuring period.

In a further embodiment, venting, temperature and/or mixing are controlled by processing means during the test to ensure that these parameters remain constant. For example, if the temperature is raised above or lowered below the initial starting temperature during the test, then the processing means is configured to adjust the temperature accordingly by decreasing or increasing power to the heating mat respectively.

It will be appreciated that different components will differ in their activity and consequently the length of the measuring period can be varied in order to take account of these discrepancies. For example, samples with low levels of activity may need a longer cycle time to ensure that small pressure changes can be clearly distinguished. In one embodiment, the measuring cycle can be varied between 30 and 6000, typically between 30 and 600 seconds using the processing means.

In one embodiment, step (d) is repeated one or more times. In a further embodiment, step (d) is repeated with identical samples (e.g. samples having the same concentration). In a yet further embodiment, step (d) is repeated with differing samples (e.g. samples having different concentrations). This embodiment provides the advantage of allowing the study of enzyme-substrate reaction kinetics (e.g. a concentration dependent analysis of enzyme-substrate reactions).

The pressure measurements may be transferred to processing means and processed simultaneously with step (d). In one embodiment, the processing means are arranged to display pressure changes numerically during a test. This numeric data can then be relayed to a computer, where manipulation allows the presentation of results in a graphical format.

In a further embodiment, the processing means is capable of full data processing, i.e. it can calculate the rate of change in pressure. In a yet further embodiment, the processing means additionally displays the real-time, pressure changes in a graphical format during the test. In a yet further embodiment, the display may also show the status of various conditions, e.g. pressure, temperature and mixing.

A result is available within a few minutes, depending upon the parameters set for the run. The user has the ability to calculate gradient of the pressure change from a selected part of the plot. The system also allows archived plots to be overlaid onto the result. The data can then be manipulated, labelled and/or time stamped.

Disclosed herein is a pressure sensing device comprising a plurality of sealable chambers and a pressure sensor which is configured, in use, to measure multiple pressure differences between any of said chambers over a specific time period.

Disclosed herein is a pressure sensing device comprising a plurality of sealable chambers and a direct pressure differential measurement sensor which is configured, in use, to directly measure the pressure differential between any of said chambers over a specific time period.

The plurality of sealable chambers may comprise one or more test chambers and a reference chamber.

The plurality of sealable chambers may comprise a test chamber and a reference chamber.

Each chamber comprises a removable receptacle. This allows the sample to be conveniently introduced and contained within the chamber during analysis. Such an arrangement provides a significant advantage of allowing sample analysis on a large scale. For example, multiple samples may be obtained and prepared simultaneously. Such samples may then either be analysed immediately in the device, or they may be stored and subsequently analysed by the device in a laboratory. The presence of sterile, single-use, disposable receptacles further eliminates the need for cleaning and/or sterilising each chamber between analyses. For example, after each analysis the receptacle can be discarded and a further receptacle can immediately be placed into the chamber. This allows the device to be either a laboratory based unit or a remote portable unit. It will also be appreciated that stirrers may also be disposable for convenience.

The receptacle may be constructed of a rigid material, suitably glass, or a mouldable or formable plastic.

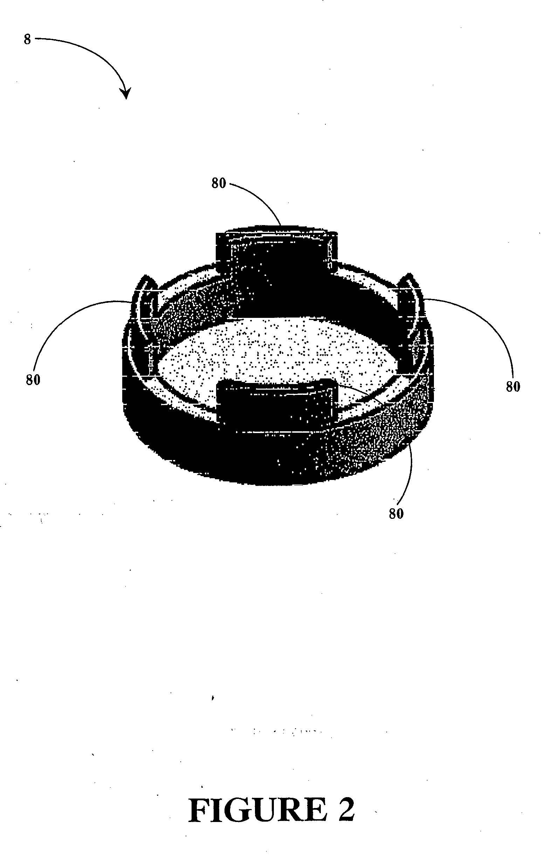

As the invention is based on gas exchange, it is desirable to ensure that there is adequate interaction between the liquid and gas phases. For this reason, the size and shape of the chamber and receptacle should be selected to allow optimum conditions for gas exchange. For example, the receptacle may be moulded substantially as a hollow cylinder, with a flat base and an open top. The top may comprise discrete vertical, upwardly extending protrusions to facilitate easier manipulation of the receptacle. The receptacle may be shaped substantially as illustrated in Figure 2. It will be appreciated that the walls of the receptacle will be arranged so that they are immediately adjacent to the walls of the chamber.

Each chamber may comprise mixing means. Gentle stirring provides the advantage of facilitating oxygen exchange between the liquid and gas phase. The mixing means may comprise one or more magnetic stirrers, such as those known in the art. Each magnetic stirrer typically comprises a magnet rotated by a motor located outside the chamber and a metallic stirrer placed within the receptacle in the chamber. The motor may be a DC motor.

The DC motors and magnets are configured to create a rotating magnetic field when each DC motor is activated, which causes the magnetic stirrers to rotate at exactly the same rate and stir the samples within the chamber. Since the magnetic coupling operates through the wall of the chamber and receptacle, there are no seals, apertures or other leakage points. Furthermore, there is no need for lubricants, which can contaminate the sample and its components. The speed at which the motors and the magnetic stirrers rotate can be varied.

The volume of the receptacle may be in the range of 500 µl to 7000 µl, suitably 3000 µl to 4000 µl where 500 to 1500 µl is the liquid (i.e. sample) phase and 4000 to 2000 µl is headspace atmosphere. The volume of the receptacle may be 3400 µl, wherein 1000 µl is the liquid (i.e. sample) phase and 2400 µl is headspace atmosphere.

The receptacle may comprise a headspace marker (e.g. a mark, line, groove or the like) to define the volume of sample required to give the optimum ratio of headspace to sample ratio.

When the volume of the receptacle is 3400 µl, the headspace marker may be present at 1000 µl.

Where the sample comprises biological components, the organisms will expire CO₂ and offset some of the pressure drop due to oxygen depletion. Therefore, each chamber may additionally comprise a suitable gas (e.g. CO₂) absorbing material to remove specific volatile compounds. The CO₂ absorber may be soda lime. The CO₂ absorber may be present in the chamber headspace.

The chambers can be sealed by one or more closure means. The closure means may comprise a lid. The chambers are designed to be gas tight when the lid is closed. It will be appreciated that the chambers will be sealed from both the atmosphere and each other.

Each chamber may additionally comprise exhaust means. This allows communication of the chamber's environment with the external environment. The exhaust means may comprise a gas-tight valve and connecting means from the valve to the chamber. The valve may be an on-off solenoid valve, which is well known to those skilled in the art. The valve may be a 3 way solenoid valve. Equilibration of the chamber pressure with ambient pressure can be carried out by opening the valves.

The device may comprise means for connecting each chamber to the pressure sensor. The connecting means may comprise gas-tight tubes. The port sizes and tubing configurations of the connecting means are important variables that have been optimised.

For example, the tubing may have a thick wall, a small diameter and a short overall length to minimise its contribution to the headspace volume. The tubing may have a bore between 1 to 2 mm, suitably 1.6 mm, and an outside diameter between 4 to 6 mm, e.g. 4.8 mm.

The direct pressure differential measurement sensor may be of any type suitable for detecting small pressure variations from about 0.1 Pa to 200 Pa. It may also be capable of providing positive or negative pressure changes relative to the reference chamber. The device will typically comprise an integrated circuit transducer which converts pressure into a voltage signal output. The pressure sensor may be an amplified pressure sensor (e.g. any pressure sensor which can be obtained from SENSORTECHNICS GmbH, Boschstr. 10, 82178 Puchheim, Germany, such as 113LP01 D, 113LP02D or 113LP05D).

The pressure sensor may be linked either directly or indirectly to processing means arranged to output the pressure differences between any of said chambers.

As used herein, the term 'processing means' refers to a computer, for example any conventional PC or Mac, desktop, laptop or pocket personal computer or PDA, or any other device capable of processing algorithms.

It will be appreciated that references to 'linked' include all known connections from processing means to peripheral hardware and include both wired and wireless connections. Such connections may therefore include universal serial bus (USB) port connections, IEEE 1394 external bus standard connections (also known as firewire, i.link and lynx), Bluetooth connections, serial connections, InfraRed (iRDA) connections or WIFI (IEEE 802.11).

The analogue electrical output may be transferred from the pressure sensor, and then relayed to the processing means. The data can be analysed to calculate the rates and significances of changes. The processing means may display pressure changes numerically during a test. This numeric data can then be relayed to a computer, where manipulation allows the presentation of results in a graphical format.

The processing means may be capable of full data processing, i.e. it can calculate the rate of change in pressure. The processing means may additionally comprise a display. This allows a user to see a real-time, graphical trend in pressure change during the test. The user then has the ability to calculate gradient of the pressure change from a selected part of the plot. The system also allows archived plots to be overlaid onto the result. The graph and the numeric slope may be printed using printing means. The result may be a number, which may be ideal for pass/fail situations in a quality control test.

The processing means may additionally comprise a real-time clock that allows results to be time and date stamped. Time/date may be displayed in a format chosen by the user. A full QWERTY keyboard also allows all samples to be readily labelled using either letters or numbers or a combination thereof. The display may also show the status of various conditions, e.g. pressure, temperature and mixing.

It will be appreciated that the device can take many forms, but should maintain precise control of temperature and control mixing effectively.

The device may additionally comprise temperature control means. This stabilises the chambers at a particular temperature, and excludes pressure variations due to temperature fluctuations. The temperature control means may comprise a thermostatic enclosure, which surrounds the chambers, wherein heat transfer occurs mostly by thermal conduction between the wall of the chamber and the receptacle. The thermostatic enclosure may be a thermal block comprising aluminium and a heating mat.

Thus, the test and reference chambers may be located in a thermal block, the temperature of which can be closely controlled (+ /- 0.01 °C) using a heating mat or by other means, such as a Peltier element. The temperature can be varied, for example from 4°C to 90°C, suitably 15°C to 60°C, to suit the characteristics of organisms of interest before the test is run. Generally, temperature will remain constant during testing.

The processing means can be arranged to monitor changes in conditions, deciding the significance of these events and altering them accordingly. The processing means is therefore able to control pressure venting via the exhaust means, temperature control means mixing means and/or the duration of each stage.

The invention may utilise one or more power sources, e.g. a DC power source or an AC power source. Examples of DC power sources include cells, batteries or any regulated power supply. The electronics of the pressure measuring device is suitably battery operated to allow for portable testing without the need for an electric mains supply. The advantage of connecting the device to processing means via an IEEE 1394 or USB port or other powered connection is that the device may utilise the power supply of the processing device rather than require a separate power source.

In view of the enhanced sensitivities of the device and the speed at which results can be obtained, it will be appreciated that the invention can be applied in a number of ways. For example, the device can either be used to detect the presence of micro-organisms within any given sample, whether in a solid phase, such as a culture gel, or liquid phase, or it may be used to monitor activity levels of a specific species. Furthermore, the device can examine the effect of an additional substance upon a micro-organism to assess whether it enhances or inhibits activity. In this way, potential nutrients, preservatives, pollutants or antibiotics can be examined for any influence on micro-organism activity levels. Finally, the device can also detect the rate at which oxygen is consumed, in solutions, aqueous emulsions and/or suspensions. These tests can be carried out on raw materials, in-process products, finished products, and the plant environment.

These applications can be used in a variety of fields including, but not limited to, the medical field, environmental field, domestic applications and a number of related industries. For example, the device is capable of testing for bacterial activity in biomedical applications. For example, the device may find particular use in hospitals and surgeries which can be easily and regularly analysed for rapid assessment and treatment of bacterial infection.

Furthermore, the invention can also be used to determine microbial activity in natural and synthetic latex, cosmetic and paint samples. Contamination is a critical quality issue for manufacturers in these industries and control using existing technologies carries significant cost as well as causing delay during the manufacturing process. Applications also exist in the food industry where contamination is a major safety concern.

Additionally, the device can also be used to monitor biocide efficacy in the control of bacterial or fungal growth in water treatment applications or in any aqueous emulsion and monitor potential toxicity of incoming effluents in water treatment plants.

Thus, Disclosed herein is the use of the device as hereinbefore defined in any of the fields or industries as described hereinbefore.

Disclosed herein is the use of the device as hereinbefore defined in monitoring biocide efficacy.

Further disclosed herein is a micro-organism detection kit which comprises a device as defined hereinbefore and instructions to use said kit in accordance with the process as hereinbefore defined.

The kit may additionally comprise one or more removable receptacles.

The invention will now be described, by way of example only, with reference to the accompanying drawings in which:
**Figure 1** shows a schematic view of the pressure sensing device according to one embodiment of the invention;
**Figure 2** shows a schematic view of the removable receptacle according to one embodiment of the invention;
**Figure 3** demonstrates the results of an analysis of biocide efficacy in the control of bacterial growth in nitrile latex; and
**Figure 4** demonstrates the results of an analysis of microbial activity in different paint samples.

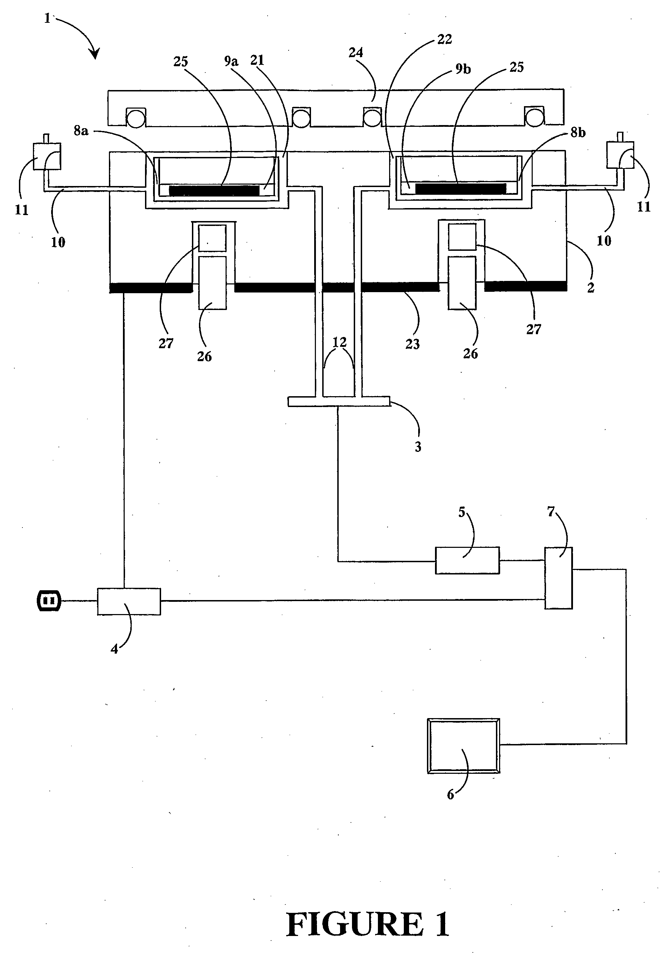

In Figure 1, a pressure sensing device, shown generally as 1, comprises a body 2, a pressure sensor 3, temperature adjusting means 4, analogue to digital signal conversion means 5, processing means 6 and an interface 7 between the device 1 and the processing means 6.

The body 2 is an aluminium thermal block which comprises two sealable chambers: a reference chamber 21 and a test chamber 22. In addition, the body 2 also comprises a heating mat 23, a sealing lid 24 a magnetic stirrer bar 25, a DC motor 26 and a magnet 27.

The DC motor 26 and magnet 27 are configured to create a rotating magnetic field when the motor 26 is activated. The rotating magnetic field therefore causes the magnetic stirrer bar 25 to revolve.

Each sealable chamber, 21 and 22, comprises a receptacle 8a and 8b, respectively, that holds a sample (e.g. reference sample 9a and test sample 9b, respectively) and a magnetic stirrer bar 25. Figure 2 shows one schematic representation of a receptacle, shown generally as 8 having cylindrical walls, a flat base and an open top. The receptacle 8 also comprises discrete vertical, upwardly extending protrusions 80 which facilitate easier manipulation of the receptacle 8.

A cylindrical gas-tight tube 10 connects each chamber 21, 22 to a solenoid valve 11, and a second gastight tube 12, connects each chamber 21, 22 to the pressure sensor 3.

The pressure sensor 3 is linked to the processing means 6 through the signal conversion means 5. The processing means 6 is also linked to the interface 7, temperature adjusting means 4, the solenoid valves 11 and the magnetic stirrers 25. In one embodiment (not shown), the interface 7 comprises a QWERTY keyboard and a display.

In use, a receptacle 8a containing a reference sample 9a is placed into the reference chamber 21, whilst a receptacle 8b containing an equal amount of test sample 9b is placed in the test chamber 22. The lid 24 is then closed and the parameters are adjusted using the processing means 6. For example, both chambers can be vented to equilibrate pressure and zero the pressure sensor 3; temperature can be set using the temperature adjusting means 4, which uniformly heats each chamber by controlling the heating mat 23 and thermal block 2; and stirring can be initiated by rotating the magnetic stirrer 25 at a uniform speed. Each sample 9a, 9b is exposed to exactly the same conditions.

The pressure sensor 3 calculates the relative pressure change between the two chambers 21, 22. Multiple measurements are made, such as 1/second, over a specific period and the resulting data is fed to the processing means 6 after being converted into a digital signal by the signal conversion means 5.

The processing means 3 can then display the real-time, pressure changes in a graphical format during analysis on the interface 7. The processing means 3 also controls the solenoid valves 11, temperature adjusting 4 means and/or the magnetic stirrers 25 during analysis to ensure that the parameters remain constant. For example, if the temperature is raised above or lowered below the initial starting temperature during analysis, then the processing means 3 adjusts the temperature accordingly by decreasing or increasing power to the heating mat 23 respectively.

A result is available within a few minutes, depending upon the parameters set for the run. The data can then be manipulated, labelled and/or time stamped using the interface 7.

### EXAMPLES

### Example 1: Detection of Biocide effect upon Bacterial Growth in Nitrile Latex

### (a) Materials Used

non-sterile latex solution (test sample);
sterilised latex solution (reference sample); and
0.2% v/v Nipacide® (a parabens or 4-hydrobenzoic acid ester biocide; obtained from Clariant UK Ltd, Horsforth, Calverley Lane, Horsforth, Leeds, West Yorkshire, LS18 4RP, UK).

### (b) Measurement Conditions

Incubation step: 300 seconds with constant stirring
Measuring cycle: 60 seconds
Measurements made: 2/second
Temperature: 30°C

### (c) Analysis of Efficacy of Biocide

1ml of the test sample was added to a test receptacle having a headspace volume of approximately 3.4ml and placed in the test chamber of a pressure sensing device as described herein. 1ml of a reference sample was likewise added to a reference receptacle also having a headspace volume of approximately 3.4ml which was then placed in the reference chamber of the same pressure sensing device.

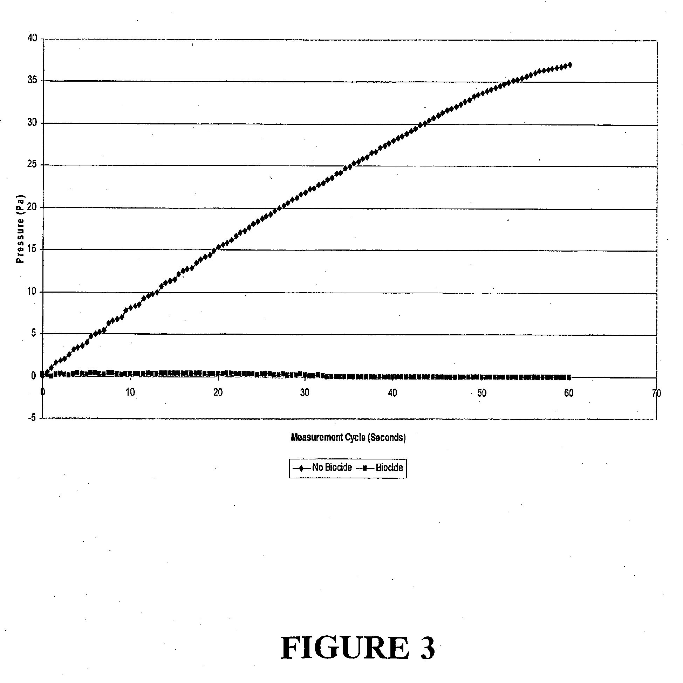

Pressure measurement was then conducted as described herein according to the parameters defined in section (b) above. The biocide was then added to the test sample and the method was repeated immediately. The results of this analysis can be seen in Figure 3 which demonstrates an immediate detection of pressure reduction. For example, a pressure reduction can be seen within just a few seconds and the pressure differential appears to be as sensitive as 1Pa.

### Example 2: Detection of microbial activity in different paint samples

### (a) Materials Used

3 non-sterile paint solutions (test samples) with the following levels of microbial contamination (each sample having been assayed by conventional microbiological techniques):
(1) paint sample 1: 10¹ cfu
(2) paint sample 2: 10² cfu
(3) paint sample 3: < 10 cfu; and
sterilised paint solution (reference sample)

### (b) Measurement Conditions

Incubation step: 300 seconds with constant stirring
Measuring cycle: 60 seconds
Measurements made: 2/second
Temperature: 30°C

### (c) Detection of Microbial Activity

1g of a test sample was added to a test receptacle having a headspace volume of approximately 3.4ml and placed in the test chamber of a pressure sensing device as described herein. 1g of the reference sample was likewise added to a reference receptacle also having a headspace volume of approximately 3.4ml which was then placed in the reference chamber of the same pressure sensing device.

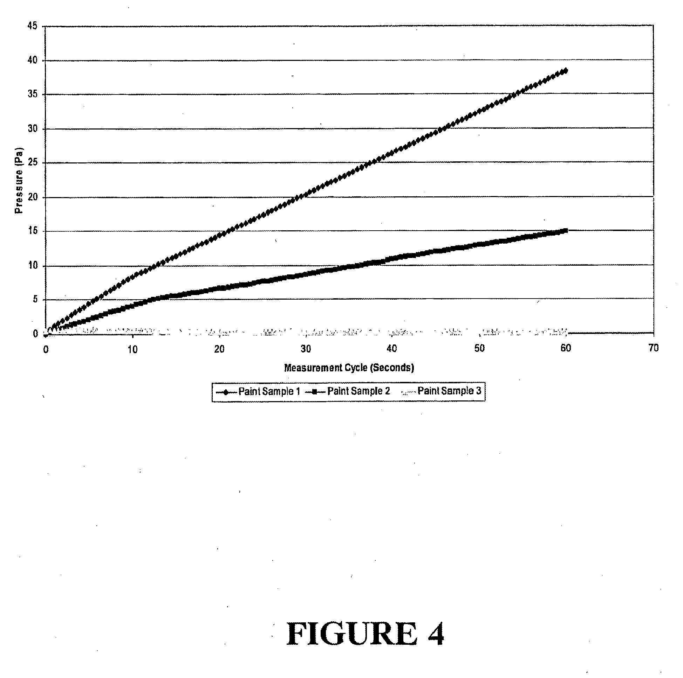

Pressure measurement was then conducted as described herein according to the parameters defined in section (b) above. The method was then repeated with a different paint test sample. The results of this analysis can be seen in Figure 4 which demonstrates a divergence in pressure difference between the three paint samples after just a few seconds, which corresponds to the level of contamination.

## Claims

1. A method for determining the presence of micro-organisms in a test sample and/or monitoring the effect of an additional substance upon a micro-organism, which comprises the steps of:
(a) adding a reference sample to a receptacle within a reference chamber, wherein said reference sample has a known level of micro-organism activity;
(b) adding the test sample to a receptacle within a test chamber, wherein said test sample has an unknown level of micro-organism activity;
(c) sealing the chambers from the external environment; and
(d) directly and continuously monitoring the pressure differential between each of said chambers by taking multiple measurements, such as 1/second, over a specific time period,
wherein said method additionally comprises one or more parameter adjustment steps, such as temperature and/or mixing adjustment, prior to step (d).

2. A method as defined in claim 1, wherein the parameter adjustment step is conducted by processing means.

3. A method as defined in claim 2, wherein temperature, and/or mixing is controlled by processing means during step (d).

4. A method as defined in claim 2 or claim 3, wherein the measuring cycle is varied between 30 and 6000 seconds, such as between 30 and 600 seconds using the processing means.

5. A method as defined in any preceding claims, wherein step (d) is repeated.

6. A method as defined in any preceding claims, wherein the pressure measurements are transferred to processing means and processed simultaneously with step (d).

## Patentansprüche

1. Verfahren zum Bestimmen der Anwesenheit von Mikroorganismen in einer Testprobe und/oder Beobachten des Einflusses einer zusätzlichen Substanz auf einen Mikroorganismus, das die folgenden Schritte beinhaltet:
(a) Geben einer Referenzprobe in einen Behälter innerhalb einer Referenzkammer, wobei die genannte Referenzprobe ein bekanntes Niveau an Mikroorganismusaktivität hat;
(b) Geben der Testprobe in einen Behälter innerhalb einer Testkammer, wobei die genannte Testprobe ein unbekanntes Niveau an Mikroorganismusaktivität hat;
(c) Abdichten der Kammern gegenüber der Außenumgebung; und
(d) direktes und kontinuierliches Beobachten des Druckdifferentials zwischen den jeweiligen genannten Kammern durch Vornehmen mehrerer Messungen, z.B. 1/Sekunde, über einen bestimmten Zeitraum,
wobei das genannte Verfahren zusätzlich einen oder mehrere Schritte zur Parameteranpassung, wie z.B. Anpassung von Temperatur und/oder Mischvorgang, vor Schritt (d) beinhaltet.

2. Verfahren nach Anspruch 1, wobei der Schritt der Parameteranpassung mit einem Verarbeitungsmittel durchgeführt wird.

3. Verfahren nach Anspruch 2, wobei die Temperatur und/oder der Mischvorgang mit einem Verarbeitungsmittel im Laufe von Schritt (d) reguliert wird/werden.

4. Verfahren nach Anspruch 2 oder Anspruch 3, wobei der Messzyklus zwischen 30 und 6000 Sekunden, z.B. zwischen 30 und 600 Sekunden, mit dem Verarbeitungsmittel variiert.

5. Verfahren nach einem der vorherigen Ansprüche, wobei Schritt (d) wiederholt wird.

6. Verfahren nach einem der vorherigen Ansprüche, wobei die Druckmessungen zum Verarbeitungsmittel transferiert und gleichzeitig mit Schritt (d) verarbeitet werden.

## Revendications

1. Procédé de détermination de la présence de microorganismes dans un échantillon d'essai et/ou de surveillance de l'effet d'une substance supplémentaire sur un microorganisme, qui comprend les étapes suivantes :
(a) addition d'un échantillon de référence dans un réceptacle dans une chambre de référence, ledit échantillon de référence contenant un microorganisme à un niveau d'activité connu ;
(b) addition de l'échantillon d'essai dans un réceptacle dans une chambre d'essai, ledit échantillon d'essai contenant un microorganisme à un niveau d'activité inconnu ;
(c) isolation hermétique des chambres de l'environnement externe ; et
(d) surveillance directe et continue de la pression différentielle entre chacune desdites chambres par la prise de mesures multiples, par exemple à raison de 1/seconde, sur une période de temps donnée,
où ledit procédé comprend en outre une ou plusieurs étapes d'ajustement de paramètres, par exemple un ajustement de la température et/ou du mélange, avant l'étape (d).

2. Procédé tel que défini à la revendication 1, dans lequel l'étape d'ajustement de paramètres utilise des moyens de traitement.

3. Procédé tel que défini à la revendication 2, dans lequel la température et/ou le mélange est contrôlé par des moyens de traitement pendant l'étape (d).

4. Procédé tel que défini à la revendication 2 ou à la revendication 3, dans lequel les moyens de traitement sont utilisés pour faire varier le cycle de mesure entre 30 et 6 000 secondes, par exemple entre 30 et 600 secondes.

5. Procédé tel que défini à l'une quelconque des revendications précédentes, dans lequel l'étape (d) est répétée.

6. Procédé tel que défini à l'une quelconque des revendications précédentes, dans lequel les mesures de la pression sont transférées aux moyens de traitement et traitées simultanément à l'étape (d).
